(19) Europäisches Patentamt
European Patent Office
Office européen des brevets

(11) **EP 2 080 747 A1**

(12) **EUROPEAN PATENT APPLICATION**

(43) Date of publication:
**22.07.2009 Bulletin 2009/30**

(51) Int Cl.:
*C07C 5/333* *(2006.01)*    *C07C 5/42* *(2006.01)*
*C07C 11/06* *(2006.01)*

(21) Application number: **09150014.0**

(22) Date of filing: **02.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **11.01.2008 US 10746 P**

(71) Applicant: **Rohm and Haas Company Philadelphia, PA 19106-2399 (US)**

(72) Inventors:
• **Benderly, Abraham Elkins Park, PA 19027 (US)**
• **Ruettinger, Wolfgang East Windsor, NJ 08520 (US)**
• **Han, Scott Lawrenceville, NJ 08648 (US)**

(74) Representative: **Kent, Venetia Katherine Rohm and Haas Europe Services ApS - UK Branch European Patent Department 4th Floor, 22 Tudor Street London EC4Y 0AY (GB)**

(54) **Supported catalyst for conversion of propane to propene and its use in a process for that conversion**

(57)    A process for the conversion of propane to propene is disclosed wherein a silica chromium catalyst composition is contacted with a propane feed stream and a carbon dioxide. The silica chromium catalyst composition is further disclosed wherein the composition, optionally, includes a promoter component.

## Description

This invention was made with Government support under Instrument No. DE-FC36-O4GO14272 awarded by the United States Department of Energy. The Government has certain rights in this invention.

The present invention pertains to a process for catalytic conversion of propane to propene, as well as catalysts suitable for use in such processes.

Well-known commercial processes for the production of monomers, such as acrylic acid and acrylonitrile, typically convert propene, by catalytic vapor phase oxidation, to the desired monomeric products. In view of the pressures exerted by competition in the industry, and the price difference between propane and propene, efforts are being made to develop processes in which propane is used as the starting material to, ultimately, produce the desired monomers at a lower overall cost.

Takehira, et al. tested the activities of various metal oxide catalysts (Cr, Ga, Ni, V, Fe, Mn and Co) supported on silicon-containing support materials, including mesoporous MCM-41, Cab-O-Sil, and silicon oxide, and found that the Cr-based catalyst supported on MCM-41 provided the best results for dehydrogenation of propane, in the presence of carbon dioxide, to form propene. (See Takehira, K.; Oishi, Y.; Shishido, T.; Kawabata, T.; Takaki, K.; Zhang, Q.; and Wang, Y., "CO2 Dehydrogenation of Propane over Cr-MCM-41 Catalyst," Studies in Surface Science and Catalysis, 2004, 153, 323-328.) While a variety of these metal oxide/support combinations have shown promise as catalyst systems for the conversion of propane to propene, there remains a need to identify systems capable of producing high yields of propene from propane. It is further desirable to identify catalyst systems capable of producing such high propene yields by endothermic dehydration. Such endothermic processes would afford the option of being coupled with processes for exothermic dehydration of propane targeting an overall zero, or very low energy usage for the resultant combined process.

We have discovered a process for catalytic conversion of propane to propene in the presence of carbon dioxide wherein the yield of propene is surprising high. Endothermic dehydrogenation is achieved using a silica chromium catalyst composition wherein chromium oxides are supported on porous silica having a surface area of at least 400 to no more than 1,200 square meters per gram, and wherein the pores of the porous silica have an average pore size of at least 20 to less than 100 Angstroms.

An aspect of the present invention is directed to process for conversion of propane to propene, the process including the steps of:

(A) contacting a propane feed stream and carbon dioxide with a silica chromium catalyst composition;
wherein the propane feed stream comprises propane; and
wherein the silica chromium catalyst composition comprises:

a) a catalytic component comprising chromium oxides present in an amount of at least 2 weight percent and no more than 15 weight percent, calculated as chromium metal equivalents based on the weight of the porous silica; and
b) a support component comprising a porous silica having:

i) plural pores having an average pore diameter of at least 20 Angstroms and less than 100 Angstroms; and
ii) a surface area of at least 400 square meters per gram and no more than 1,200 square meters per gram; and

(B) converting the propane to propene, in the presence of the carbon dioxide and the silica chromium catalyst composition.

Used herein, the following terms have these definitions:

The words "a" and "an" as used in the specification mean "at least one", unless otherwise specifically stated.

"Range". Disclosures of ranges herein take the form of lower and upper limits. There may be one or more lower limits and, independently, one or more upper limits. A given range is defined by selecting one lower limit and one upper limit. The selected lower and upper limits then define the boundaries of that particular range. All ranges that can be defined in this way are inclusive and combinable, meaning that any lower limit may be combined with any upper limit to delineate a range. For example, if ranges of 60 to 120 and 80 to 110 are recited for a particular parameter, it is understood that the ranges of 60 to 110 and 80 to 120 are also contemplated. Additionally, if minimum range values of 1 and 2 are recited, and if maximum range values of 3, 4, and 5 are recited, then the following ranges are all contemplated: 1 to 3, 1 to 4, 1 to 5, 2 to 3, 2 to 4, and 2 to 5.

It will be appreciated by those skilled in the art that changes could be made to the suitable methods and compositions specifically described herein without departing from the broad inventive concept thereof. It is understood, therefore, that this invention is not limited to the particular suitable methods and compositions disclosed, and that recitation thereof is intended to additionally cover modifications within the spirit and scope of the present invention as disclosed herein and as defined by the appended claims.

Carbon dioxide (interchangeably "carbon dioxide gas") is capable of participating as an oxidant in the catalytic dehydrogenation of propane, combining with hydrogen atoms removed from the propane to produce a by-product compound containing at least one hydrogen atom removed from propane and at least one atom previously contained in the carbon dioxide.

A "silica chromium catalyst composition" is a composition including a "catalytic component", a "support component", and, optionally, a "promoter component".

A "catalytic component" is a component including chromium oxides, wherein the catalytic component is capable of catalyzing endothermic dehydrogenation of propane to form propene. The chromium is typically present as Cr(III) during the endothermic dehydrogenation which converts propane to propene, but may further be present in other oxidation states such as Cr(IV).

A "support component" is a component including "porous silica", the porous silica including: plural pores having an average pore diameter of at least 20 Angstroms to less than 100 Angstroms; and a surface area of at least 400 $m^2$/g and no more than 1,200 $m^2$/g.

A "promoter component" is a component including a "catalyst promoter".

A "catalyst promoter" is a material which, when included in the silica chromium catalyst composition of the present invention, changes the silica chromium catalyst composition, typically augmenting its catalytic efficiency, or catalyst lifetime, for converting propane to propene.

A "diluent," is any material which is substantially inert, i.e., does not participate in, is unaffected by, and/or is inactive, in the particular reaction of concern. For example, nitrogen gas is inert in oxidative dehydrogenation reactions that produce propene from propane.

The term "propane weight fraction conversion" ("propane w. f. conversion") is defined as the difference between 1 and the dividend determined by dividing the moles of propane contained in a feed stock for an endothermic dehydrogenation into the moles of propane consumed in that dehydrogenation. The "propane percentage conversion" ("propane % convention") is defined as the propane weight fraction conversion multiplied by 100%. Values for moles of propane contained in the feed stock and moles of propane contained in the product stream after the chemical reaction may be determined analytically.

$$\text{propane weight fraction conversion} \ = \ \left( 1 \ - \ \frac{\text{moles of propane unconverted}}{\text{moles of propane in feedstock}} \right)$$

$$\text{propane \% conversion} \ = \ (\text{propane weight fraction conversion}) \ \text{X} \ 100\%$$

The term "propene weight fraction selectivity" ("propene w. f. selectivity") is defined as the difference between the moles of propane in the feedstock and the moles of propane remaining unconverted after the chemical reaction divided into the moles of propene formed during the chemical reaction. The "propene percentage selectivity" ("propene % selectivity") is defined as the propene weight fraction selectivity multiplied by 100%. The value for moles of propene contained in the product stream after the chemical reaction may be determined analytically.

$$\text{propene weight fraction selectivity} \ = \ \left( \frac{\text{moles of propene formed}}{(\text{moles propane in feedstock}) - (\text{moles propane unconverted})} \right)$$

$$\text{propene \% selectivity} \ = \ (\text{propene weight fraction selectivity}) \ \text{X} \ 100\%$$

The term "propene weight fraction yield" ("propene w. f. yield") is defined as the propane weight fraction conversion multiplied by the propene weight fraction selectivity. The "propene percentage yield" ("propene % yield") is defined as the propene weight fraction yield multiplied by 100%.

$$\text{propene weight fraction yield} = (\text{propane w. f. conversion}) \times (\text{propene w. f. selectivity})$$

$$\text{propene \% yield} = (\text{propene weight fraction yield}) \times 100\%$$

The term "carbon dioxide-containing gas" means any gas which includes from 0.01% up to 100% carbon dioxide. Non-limiting examples of carbon dioxide-containing gas include: carbon dioxide enriched air, pure carbon dioxide, and mixtures of carbon dioxide with inert gases such as nitrogen and argon.

"Oxidative dehydrogenation" means a chemical reaction in which a hydrocarbon and oxygen are reacted to result in removal of one or more hydrogen atoms from the hydrocarbon to produce oxidation products. Thus oxidative dehydrogenation requires an oxygen-containing gas or a gaseous oxygen-containing compound to provide the required oxygen.

"Dehydrogenation" is a chemical reaction in which one or more hydrogen atoms are removed from a chemical moiety to form a product moiety containing unsaturation not present in the original chemical moiety. Herein, "dehydrogenation" refers to the removal of two hydrogen atoms from propane to form propene having a double bond not present prior to the dehydrogenation.

"Endothermic dehydrogenation" is a dehydrogenation reaction accompanied by consumption of heat such that the reaction requires heat to be supplied from a source external to the reaction.

"Exothermic dehydrogenation" is a dehydrogenation reaction accompanied by evolution of heat such that the reaction requires heat to be removed by a cooling means.

The "catalyst promoter" of the present invention includes oxides of a metal selected from vanadium (V); silver (Ag); Cerium (Ce) and other lanthanides such as La, Pr, Nd, and Gd; Ti; Zr; V; Nb; Mo; W; Zn; and combinations thereof.

A "propane feed stream" ("feed stream") is a gaseous stream including propane. A propane feed stream may be pure or substantially pure propane. Alternatively, the propane feed stream may further include one or more components of a recycled propene product stream such as, for example, diluent, propene, propane dehydrogenation by-products, steam, carbon monoxide (CO), and combinations thereof.

"Propane dehydrogenation by-products" are by-products formed during a reaction converting propane to propene ("propane dehydrogenation reaction") wherein at least a portion of the propane that has been consumed has not been converted to propene. Typical propane dehydrogenation by-products include, but are not limited to: methane, ethylene, ethane, acetylene, and combinations thereof.

An "endothermic reaction zone" is a reaction zone in which the endothermic dehydrogenation is accomplished. A "dehydrogenation reactor" of the present invention includes an endothermic reaction zone.

The "support component" of the present invention includes "porous silica" having plural pores. The plural pores of the porous silica of the present invention have an average pore diameter of: at least 20, at least 25, or at least 30 Angstoms (Å); and less than 100, no more than 80, no more than 70, or no more than 60 Å. The plural pores of the porous silica of the present invention have a surface area of: at least 400, or at least 450 square meters/gram, (m²/g), and no more than 1,200, no more than 1,000, no more than 800, or no more than 700 m²/gram, based on the weight of the porous silica. The support component can be, for example, in the shape of beads, pills, pellets, cylinders, extrudates (e.g., trilobes), tubes, spherical particles, irregularly shaped particles, regularly shaped monoliths, and irregularly shaped monoliths.

The "catalytic component" of the present invention includes chromium as chromium oxides. Chromium oxides are present in the "silica chromium catalyst composition" in an amount of: at least 2, at least 3, or at least 4 weight percent; and no more than 15, no more than 12, or no more than 10 weight percent, calculated as chromium metal equivalents based on the weight of the support component.

The amount of the catalyst promoter of the present invention is selected to optimize the efficiency and lifetime of the catalytic component. When present, the catalyst promoter is present in the silica chromium catalyst composition in an amount of: equal to or greater than 0.01, at least 0.05, at least 0.5, at least 1, or at least 2 weight percent; and no more than 10, no more than 8, no more than 5, or no more than 2.5 weight percent, calculated as metal equivalents based on the weight of the support component. Vanadium oxides are present in the silica chromium catalyst composition in an amount of: equal to or greater than 0, at least 0.5, at least 1, or at least 2 weight percent; and no more than 10, no more than 8, or no more than 5 weight percent, calculated as vanadium metal equivalents based on the weight of the support component. Silver oxides are present in the silica chromium catalyst composition in an amount of: equal to or greater than 0, at least 0.01, at least 0.05, or at least 0.1 weight percent; and no more than 2, no more than 1, or no more than 0.5 weight percent, calculated as silver metal equivalents based on the weight of the support component.

The preparation of the silica supported chromium based catalyst typically includes a step of calcining. The skilled practitioner will recognize calcining as a step which is performed on a support component which has been loaded with a solution including a catalyst precursor. In the step of calcining, the support component loaded with the catalyst precursor solution is heated to evaporate the solvent. For example, when the solvent is water, it is advantageous to first heat at a temperature near, but below the boiling point of water, followed by prolonged drying at a temperature near, but above the boiling point of water. The step of calcining is accomplished by further elevating the temperature to a temperature at which the catalyst precursor (e.g., a metal salt) is converted, in the presence of oxygen, to the corresponding metal oxides. Typically, the calcining temperature will be in the range of 450 to 750 °C. The calcining step of the present invention is used to convert a chromium catalyst precursor (typically a metal salt) to a catalytic component including chromium oxides. The calcining step may be accomplished at a single temperature and hold period or at multiple temperatures and/or hold periods. The preparation of the silica chromium catalyst composition may further include multiple catalyst component /promoter component loading steps and/or multiple calcining steps.

The skilled practitioner will also recognize that the support component may be further loaded with a solution including a promoter precursor prior to evaporation of solvent and calcining. The solution including the catalyst precursor may be the same as the solution including the promoter precursor. Alternatively, the solution including the catalyst precursor and the solution including the promoter precursor can be two different solutions. In a further suitable alternative: a portion of the promoter precursor may be combined in solution with all or a portion of the catalyst precursor; or a portion of the promoter precursor may be combined in solution with all or a portion of the catalyst precursor.

In the process of the present invention, the endothermic reaction zone is provided with the silica chromium catalyst composition of the present invention. The propane feed stream, the carbon dioxide gas, and any diluent may be introduced to the endothermic reaction zone separately, or portions or all of each may be combined. The propane feed stream and the carbon dioxide gas are contacted with the silica chromium catalyst composition at a reaction temperature and reaction time affording efficient conversion of propane to propene. The "propene product stream" thus produced includes propene, and may further include unreacted propane, methane, ethylene, unreacted carbon dioxide, carbon monoxide, water vapor, and molecular hydrogen, as well as side reaction products such as methane, ethylene, and ethane.

The subsequent disposition of the propene product stream will vary depending upon its intended use. For example the propene product stream may be used without modification in an additional step, such as: preparation of a derivative of propene, examples of which are acrylic acid, esters of acrylic acid, acrylonitrile, or combinations thereof; or recycle as all or a portion of the propane feed stream for another oxidative dehydrogenation of propane in the endothermic reaction zone. The skilled practitioner will further recognize that the propene product stream may be subjected to a step of "selective partitioning" in which all or a portion of one or more non-propene components is selectively removed. The step of selective partitioning may be used to prepare purified propene useful in subsequent conversion to derivatives of propene, including those just described. The step of selective partitioning may further be used to concentrate unreacted propane which can then be recycled as all or part of another propane feed stream for oxidative dehydrogenation.

Suitable "diluents" include, but are not limited to: nitrogen; noble gases such as helium and argon; and steam.

The "dehydrogenation feed composition" of the present invention includes propane in an amount of: at least 5, at least 10, or at least 15 percent by volume; and no more than 70, no more than 40, or no more than 30 percent by volume, based on the total volume of the dehydrogenation feed composition. The dehydrogenation feed composition of the present invention further includes carbon dioxide in an amount of: at least 10 or at least 20 percent by volume; and no more than 80 or no more than 60 percent by volume, based on the total volume of the dehydrogenation feed composition. The dehydrogenation feed composition may still further include diluent in an amount of: equal to or greater than 0, at least 1, or at least 5 percent by volume; and no more than 50, no more than 40, or no more than 20 percent by volume, based on the total volume of the dehydrogenation feed composition. Suitable dehydrogenation feed compositions may yet further included propane dehydrogenation by-products in an amount of: equal to or greater than 0, at least 1, or at least 2 percent by volume; and no more than 20, no more than 10, or no more than 5 percent by volume, based on the total volume of the propane feed composition. Propane dehydrogenation by-products may, typically, be present in the dehydrogenation feed composition when all or a portion of the propene product stream is recycled.

The "support component" of the present invention includes "porous silica" having plural pores. The plural pores of the porous silica of the present invention have an average pore diameter of: at least 20, at least 25, or at least 30 Angstoms (Å); and less than 100, no more than 80, no more than 70, or no more than 60 Å. The plural pores of the porous silica of the present invention have a surface area of: at least 400, or at least 450 square meters/gram, ($m^2$/g), and no more than 1,200, no more than 1,000, no more than 800, or no more than 700 $m^2$/gram, based on the weight of the porous silica. The support component can be, for example, in the shape of particles (for example, tablets and extrudates), regularly shaped monoliths, and irregularly shaped monoliths.

The silica chromium catalyst composition may be prepared by any suitable method known in the art. The support component may be modified, stabilized, or pretreated in order to achieve the proper structural stability desired for sustaining the operating conditions under which the catalysts will be used. The support component can be in the shape of tablets, extrudates, monoliths, particles, honeycombs, rings, and others. Where the support is in the form of particles, the shape of the particles is not particularly limited and may include granules, beads, pills, pellets, cylinders, trilobes, spheres, irregular shapes, etc. The catalytic component can be applied to the support component by any method known to the art including, for example, incipient wetness impregnation, chemical vapor deposition, hydrothermal synthesis, salt melt method, or co-precipitation. When a promoter component is to be included in the silica chromium catalyst composition, that promoter component can also be applied to the support component by any method known to the art including, for example, incipient wetness impregnation, chemical vapor deposition, hydrothermal synthesis, salt melt method, or co-precipitation. The catalytic component and, optionally, the promoter component may be applied to the support component at any time including, but not limited to, before or after the step of calcining. When a promoter component is used, all or a portion of the promoter component may be mixed with all or a portion of the catalytic component, prior to or during application to the support component.

One method of loading the catalytic component and, optionally, the promoter component onto the support component is the "incipient wetness process". The incipient wetness process is a process for gradually loading a solution of a catalytic component, or precursor to a catalytic component, (and, optionally, a promoter component, or precursor to a promoter component) onto a support component until the support component becomes saturated with the solution and begins to exhibit wetness at its surface. The first appearance of such wetness is termed "incipient wetness."

When the support component is a monolith, that monolith includes porous silica and is any unitary piece of continuous manufacture, such as, for example, pieces of porous silicon oxide, silica foams, or silica honeycomb structures. The monolith can further be structured as a silicon oxide "honeycomb" straight channel extrudate or other monolith having longitudinal channels or passageways permitting high space velocities with a minimal pressure drop. The monolith can further be supported on a "monolithic substructure", such as a refractory oxide "honeycomb" straight channel extrudate or monolith, made of cordierite or mullite, or other configuration having longitudinal channels or passageways permitting high space velocities with a minimal pressure drop. It is known in the art that, if desired, a reaction zone may include two or more monoliths stacked upon one another.

Furthermore, the catalytic component may be deposited as one or more wash coats on a monolithic support component by methods known to the skilled practitioner. Additionally, the catalytic component may be combined with a monolithic support component by depositing the support component on a monolithic substructure as one or more wash coats and, successively, impregnating the support component wash coat with the catalytic component and, optionally, with a promoter component.

Monolithic supports may include stabilized zirconia (PSZ) foam (stabilized with Mg, Ca or Y), or foams of $\alpha$-alumina, cordierite, ceramics, titania, mullite, zirconium-stabilized $\alpha$-alumina, or mixtures thereof. Monolithic supports may also be fabricated from metals and their alloys, such as, for example, aluminum, steel, fecralloy, hastalloy, and others known to persons skilled in the art. Additionally, other refractory foam and non-foam monoliths may serve as satisfactory supports. The promoter metal precursor and any base metal precursor, with or without a ceramic oxide support forming component, may be extruded to prepare a three-dimensional form or structure such as a honeycomb, foam or other suitable tortuous-path or straight-path structure.

The dehydrogenation reactor of the present invention may be any suitable reactor known in the art including, but not limited to, a batch reactor, a stirred tank reactor, a continuous stirred tank reactor (CSTR), a tubular reactor, a shell-and-tube heat exchanger reactor, a multiple-pass reactor, a reactor having microchannels, a short contact time reactor, a catalytic fixed bed reactor, or a reactor having a combination of the foregoing features. The dehydrogenation reactor may include a single endothermic dehydrogenation zone or multiple endothermic dehydrogenation zones. Further, each reaction zone may optionally include one or more sub-zones, each of which may differ, for example, in operating temperature, silica chromium catalyst composition, or catalytic component concentration. Furthermore, the silica chromium catalyst compositions may be configured in their respective reaction zones in any suitable arrangement including, but not limited to, a fixed bed, a fluidized bed, and spouted bed. All such configurations are well known in the art.

Suitable operating conditions for endothermic dehydrogenation of propane are generally known by persons of ordinary skill in the art and are applicable to operation of the endothermic dehydrogenation reactor. For example, the dehydrogenation feed composition may be supplied to the endothermic dehydrogenation reactor in a single stream, or as separate constituent streams. For example: the propane feed stream, the carbon dioxide, and the diluent may each be fed as a separate stream; all or a portion of each of the propane feed stream and the diluent may be combined before being fed to the reactor; all or a portion of each of the propane feed stream and the carbon dioxide may be combined before being fed to the reactor; all or a portion of each of the carbon dioxide and the diluent may be combined before being fed to the reactor; or all or a portion of each of the propane feed stream, the carbon dioxide, and the diluent may be combined before being fed to the reactor. The dehydrogenation feed composition is typically fed at a total "gas hourly space velocity" ("GHSV") of at least 500, at least 1,000, or at least 5,000 hr$^{-1}$; and no more than 100,000, no more than 50,000, or no more than 20,000 hr$^{-1}$. The reaction pressure is typically: at least 0.1, at least 0.5, or at least 1 atmosphere;

and no more than 20, no more than 10, or no more than 5 atmospheres. The reaction temperature is typically: at least 300, at least 450, at least 600, at least 620 °C; and no more than 900°C, no more than 700, or no more than 660 °C. The contact time between dehydrogenation feed composition silica chromium catalyst composition is typically: at least 0.03, at least 0.05, or at least 0.1 second; and no more than 10, no more than 8, no more than 5, or no more than 1 second. The molar ratio of propane to carbon dioxide supplied to the reactor is: at least 0.1, at least 0.2, or at least 0.5; and no more than 10, no more than 5, or no more than 2.

The practitioner will recognize that the yield of the endothermic dehydrogenation of propane of the present invention may vary depending upon such influences as, for example, dehydrogenation reactor design, dehydrogenation reactor composition, ratios of reaction components, residence time, and changes in efficiency of the silica chromium catalyst composition as a function of time on stream. However, the "fresh propene % yield" (i.e., the propene % yield determined after 10 minutes of operation) is typically: at least 25, or at least 30 percent; and no more than 60, or no more than 50 percent, wherein the propene % yield is the molar ratio of moles of propene produced divided by moles of propane fed to the reactor multiplied by 100%.

**Experimental Examples.** Some embodiments of the invention will now be described in detail in the examples. The following porous silica materials, shown in Table 1, are used in the examples.

**Table 1. Porous silica used at supports in the examples.**

| Porous Silica | Pore diameter, Å | Pore volume, cc/g | Surface area, $m^2/g$ | Source (Name and Address) |
|---|---|---|---|---|
| Merck 10181 silica gel | 40 | 0.68 | 675 | Aldrich Chemicals, Milwaukee, WI |
| Merck 10184 silica gel | 100 | | 300 | Aldrich Chemicals, Milwaukee, WI |
| Davisil 636 silica gel | 60 | 0.75 | 480 | Aldrich Chemicals, Milwaukee, WI |
| Davisil 646 silica gel | 150 | 1.15 | 300 | Aldrich Chemicals, Milwaukee, WI |
| W.R. Grace grade 12 | 22 | 0.43 | 800 | Aldrich Chemicals, Milwaukee, WI |
| W.R. Grace grade 62 | 150 | 1.15 | 300 | Aldrich Chemicals, Milwaukee, WI |
| W.R. Grace grade 923 | 30 | 0.43 | 550 | Aldrich Chemicals, Milwaukee, WI |
| GFS item 2827 | 60 | | 500 | GFS Chemicals Inc., Powell, OH |
| SIPERNAT™ silica | 202 | | 365 | Evonik Industries |
| Norpro 61137 | 11 | 0.6[a] | 156 | Saint-Gobain NorPro Corp., Akron, OH |
| a) Mercury (Hg) pore volume. | | | | |

**Test Methods**

**Determination of porosity.** Pore diameter, pore volume, and surface area for the porous silica utilized as the support component in the experimental section were provided by the commercial suppliers (see Table 1). Typically, information related to porosity is analytically determined by nitrogen adsorption according to methods disclosed by Brunauer, et al., J. Am. Chem Soc. 60, 309 (1938).

**Example 1. Preparation of propene from propane catalyzed by chromium-based catalysts on silica supports.**

**Preparation of silica supported chromium nitrate precursor.** The porous silica was impregnated to incipient wetness with an aqueous solution of $Cr(NO_3)_3$, then dried 8 hours at 80 °C, followed by 8 hours at 120°C.

**Single stage calcining procedure.** The dried silica supported chromium nitrate precursor was calcined at 650 °C for 2 hours in air in a static muffle furnace.

**Analysis for the amount of chromium oxides in calcined silica supported chromium based catalyst.** The amount of chromium oxides contained in the calcined silica chromium catalyst composition was determined by ASTM method in which chromium is leached with acid and its amount is determined by ICP. The $Cr_2O_3$ loading in the calcined silica chromium catalyst composition is calculated based on the amount of Cr-nitrate solution added in the impregnation step, and is reported as [(weight Cr2O3)/(wt catalyst)] X 100).

**Two stage calcining procedure.** After a first impregnation with aqueous $Cr(NO_3)_3$, the silica supported chromium nitrate precursor was calcined at 500°C. An amount of a second aliquot of aqueous $Cr(NO_3)_3$ solution was prepared. The amount of $Cr(NO_3)_3$ contained in that solution was calculated so that the weight of chromium in the final calcined silica supported chromium based catalyst would be 10 percent by weight, based on the weight of the calcined silica chromium catalyst composition. The second aliquot of aqueous $Cr(NO_3)_3$ solution was then added to the intermediate calcined silica chromium catalyst composition and allowed to impregnate that intermediate composition. The impregnated intermediate composition was then dried 8 hours at 80 °C, followed by 8 hours at 120 °C, and then calcined at 650 °C for 2 hours to form the final calcined silica chromium catalyst composition. The calcined silica chromium catalyst composition is a powder.

**Preparation of propene from propane.** A fritted quartz tube having an inside diameter of 10 mm is filled with 2.1g calcined silica chromium catalyst composition powder and heated to 630°C in a flow of nitrogen gas. The content of the gas is then adjusted to: 10 volume percent propane, 50 volume percent $CO_2$, and 40 volume percent nitrogen, and the gas flow rate is set at 225 ml/min. The "fresh yield" is recorded after 10 minutes on stream. Table 2 lists characteristics of catalysts, the silica supports from which they were made, and "fresh yields" for Examples 1-1 through 1-5.

**Table 2. Example 1 supports, catalysts, and results of propane to propene conversion.**

| Example Number | Support component (porous silica) | | | | Silica chromium catalyst component | | Propene % yield, fresh[a] |
|---|---|---|---|---|---|---|---|
| | Type | Pore diam., Å | Pore volume, cc/g | Surface area, $m^2/g$ | Chromium oxides(s) as chromium weight % | Surface area, $m^2/g$ | |
| 1-1 | 10181 silica gel | 40 | 0.68 | 675 | 10 | 353 | 33 |
| 1-2 | 636 Davisil silica gel | 60 | 0.75 | 480 | 10 | 407 | 33 |
| 1-3 | Aldrich grade 923 | 30 | 0.43 | 550 | 10 | 327 | 49 |
| 1-4 | GFS item 2827 | 60 | | 500 | 10 | 386 | 30 |
| 1-5 | grade 12 silica gel | 22 | 0.43 | 800 | 10 | 319 | 27 |
| a) "fresh yield" is the yield recorded after 10 minutes of operation | | | | | | | |

**Example C. Comparative examples of the preparation of propene from propane catalyzed by silica chromium catalyst compositions.** Examples C were prepared using methods identical to those of Examples 1. That is, the methods utilized to prepare the silica chromium catalyst compositions of Examples 1-1 through 1-5 and Examples C-1 through C-5 were identical. Further, the method utilized to prepare propene from propane in Examples 1-1 through 1-5 and Examples C-1 through C-5 were identical. **Table 3** lists characteristics of silica chromium catalyst component, the support component from which they were made, and "fresh yields" for Examples C-1 through C-5.

**Table 3. Comparative supports, catalysts, and results of propane to propene conversion.**

| Example Number | Support component (porous silica) | | | | Silica chromium catalyst component | | Propene % yield, fresh[a] |
|---|---|---|---|---|---|---|---|
| | Type | Pore diam., Å | Pore volume, cc/g | Surface area, $m^2/g$ | Chromium oxides(s) as chromium weight % | Surface area, $m^2/g$ | |
| C-1 | 10184 silica gel | 100 | | 300 | 10 | 325 | 23 |
| C-2 | 646 Merck silica gel | 150 | 1.15 | 300 | 10 | | 11 |
| C-3 | Aldrich grade 62 | 150 | 1.15 | 300 | 10 | 264 | 20 |
| C-4 | SIPERNAT™ silica | 202 | 2.24 | 365 | 10 | | 10 |
| C-5 | Norpro 61137 | 11 | 0.6[b] | 156 | 10 | 137 | 14 |
| a) "fresh yield" is the yield recorded after 10 minutes of operation<br>b) Mercury (Hg) pore volume. | | | | | | | |

## Claims

1. A process for conversion of propane to propene, the process comprising the steps of:

   (A) contacting a propane feed stream and carbon dioxide with a silica chromium catalyst composition; wherein the propane feed stream comprises propane; and wherein the silica chromium catalyst composition comprises:

   a) a support component comprising a porous silica having:

   i) plural pores having an average pore diameter of at least 20 Angstroms and less than 100 Angstroms; and
   ii) a surface area of at least 400 square meters per gram and no more than 1,200 square meters per gram; and

   b) a catalytic component comprising chromium oxides present in an amount of at least 2 weight percent and no more than 15 weight percent, calculated as chromium metal equivalents based on the weight of the porous silica; and

   (B) converting the propane to propene, in the presence of the carbon dioxide and the silica chromium catalyst composition.

2. The process of claim 1, wherein the average pore diameter is at least 25 Angstroms and no more than 70 Angstroms.

3. The process of claim 1, wherein the surface area is at least 450 square meters per gram and no more than 800 square meters per gram.

4. The process of claim 1, wherein the silica chromium catalyst composition further comprises a promoter component comprising a catalyst promoter selected from vanadium oxides, silver oxides, cerium oxides, molybdenum oxides, zinc oxides, zirconium oxides, and combinations thereof.

5. The process of claim 4, wherein the catalyst promoter is present in an amount of at least 0.1 weight percent and no more than 5 weight percent, calculated as metal equivalents based on the weight of the support component.

**6.** The process of claim 1, wherein the converting of propane to propene is endothermic.

Europäisches
Patentamt
European
Patent Office
Office européen
des brevets

**EUROPEAN SEARCH REPORT**

Application Number

EP 09 15 0014

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | TAKEHIRA K ET AL: "CO2 dehydrogenation of propane over Cr-MCM-41 catalyst" STUDIES IN SURFACE SCIENCE AND CATALYSIS, ELSEVIER SCIENCE B.V., AMSTERDAM, vol. 153, 1 January 2004 (2004-01-01), pages 323-328, XP009116376 ISSN: 0167-2991 * table 1 * * figure 3 * ----- | 1-6 | INV. C07C5/333 C07C5/42 C07C11/06 |
| X | LIU ET AL: "Mesoporous silica-supported chromium catalyst: Characterization and excellent performance in dehydrogenation of propane to propylene with carbon dioxide" CATALYSIS COMMUNICATIONS, ELSEVIER SCIENCE, AMSTERDAM, NL, vol. 8, no. 3, 1 March 2007 (2007-03-01), pages 565-570, XP022134694 ISSN: 1566-7367 * paragraph [02.1] * * paragraph [03.1] * * table 1 * ----- | 1-6 | |

TECHNICAL FIELDS
SEARCHED    (IPC)

C07C

The present search report has been drawn up for all claims

| Place of search | Date of completion of the search | Examiner |
|---|---|---|
| Munich | 8 June 2009 | Götz, Gerhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Takehira, K. ; Oishi, Y. ; Shishido, T. ; Kawabata, T. ; Takaki, K. ; Zhang, Q. ; Wang, Y.** CO2 Dehydrogenation of Propane over Cr-MCM-41 Catalyst. *Studies in Surface Science and Catalysis,* 2004, vol. 153, 323-328

- **Brunauer et al.** *J. Am. Chem Soc.,* 1938, vol. 60, 309